# EUROPEAN PATENT SPECIFICATION

(11) **EP 0 332 023 B1**
(45) Date of publication and mention of the grant of the patent: **04.08.1993**
(21) Application number: 89103483.7
(22) Date of filing: 28.02.1989
(51) Int. Cl.: C12N 1/04

(54) **Spray drying of microorganisms having ice nucleating activity**
Sprühtrocknung von Mikroorganismen mit Eiskeime bildender Aktivität
Séchage par pulvérisation de microorganismes ayant une activité de nucléation de la glace

(30) Priority: 07.03.1988 US 164591
(43) Date of publication of application: 13.09.1989
(73) Proprietor: GENENCOR INTERNATIONAL, INC., South San Francisco, CA 94080 (US)
(72) Inventor: Lindsey, Carole Beth c/o Eastman Kodak Company, Rochester New York 14650 (US)
(74) Representative: Brandes, Jürgen, Dr.

(56) References cited:
- EP-A- 0 063 438
- US-A- 4 706 463
- BIOLOGICAL ABSTRACTS, vol. 77, 1984, abstract no. 56567, Biological Abstracts Inc., Philadelphia, PA, US; T. MAKINO: "Ice-nucleation activity of bacteria isolated from gemmmisphere of tea trees", & ANN PHYTOPATHOL. SOC. JPN 49(1): 32-37. 1983
- J. FERMENT. TECHNOL., vol. 65, no. 6, 1987, pages 693-697; H. OBATA et al.: "Ice-nucleating activity of pseudomonas fluorescens"
- J. AMER. SOC. HORT. SCI., vol. 107, no. 1, 1982, pages 123-125; J.A. ANDERSON et al.: "Frost injury of tender plants increased by pseudomonas syringae van hall"
- J. APPLIED METEOROLOGY, no. 17, July 1978, pages 1049-1053, American Meteorological Society; L.R. MAKI et al.: "Bacteria as biogenic sources of freezing nuclei"

## Description

The present invention relates to a method for the recovery, in dried form, of microorganisms that have ice nucleating activity from a medium containing the microorganism.

In US Patent 4,200,228 there is disclosed a method for the making of snow whereby microorganisms are included in droplets that are sprayed into the air. The microorganisms that are used are of the type which are known to promote ice nucleation. As a result, snow can be made at temperatures that are much higher than are ordinarily possible. A typical microorganism that is useful in this process is a Pseudomonad and particularly Pseudomonas syringae.

It is apparent that if this process is to be used on any scale, large amounts of microorganisms are needed. Further, it is desirable that the microorganism be obtained in a dry form so as to facilitate the storage handling and transport of the material.

The growth conditions for microorganisms that have ice nucleating activity are known in the art. For example, in Maki and Willoughby, Bacteria as Biogenic Sources of Freezing Nuclei, J. Applied Meteorology 17 1049-1053 it is disclosed that the microorganisms such as Pseudomonas syringae are grown in Koser citrate broth at a temperature below 20°C, i. e. 5°C. It is also stated that if the cultures are grown at a temperature above 20°C, very few freezing nuclei are produced.

As far as the recovery process is concerned, this reference discloses that concentrated cultures that had been treated with formalin were freeze dried. No details are given.

In another reference, the microorganisms are grown on a tryptone-yeast extract-glycerol medium which would have a pH of 7.0. (Koxloff, Schofield and Lute, Ice Nucleating Activity of Pseudomonas syringae and Erwinia herbicola, J. Bacter. 153 pages 222-231 (1983)) In this reference, the microorganisms are not recovered in dry form and the suspensions are tested directly for activity. It is noted that the ice nucleating activity is not stable in the suspension and decreases overnight.

If the known procedures are used for the production of large volumes of the microorganisms, less than the desired ice nucleating activity is obtained. Even where the fermentation produces a high activity in the fermentation suspension, much of the activity is lost during the drying of large volumes of the material. The end result is a process that is not capable of producing commercial quantities of microorganism at reasonable cost.

In response to this need, there was provided a process for the recovery of the ice nucleating microorganisms that provided for the preservation of most of the ice nucleating activity (INA). That process is disclosed and claimed in US Patent 4,706,463 issued November 17, 1987. In that process, the medium is first cooled and then concentrated. After concentration, the medium is frozen by running the concentrate into a cryogenic fluid. The pellets that result are then freeze dried.

The process that is described in the above patent is quite expensive to operate. Large volumes of expensive cryogenic fluid are needed. In addition, the freeze drying step is more expensive than desired. Therefore, the problem to be solved by the present invention is to provide a less expensive alternative to the cryogenic fluid-freeze drying process previously known.

The present invention provides a method for the recovery of a microorganism which has ice nucleating activity from a fermentation medium. The method involves the same cooling and concentration steps of the previous method but the concentrate is then spray dried under certain conditions. Thus, in accordance with the present invention, the method comprises the steps of:
a) bringing the temperature of said medium to a temperature of 15°C or less,
b) forming a concentrate of said microorganism preferably having a water content of 15-27%, while maintaining the temperature of 15°C or less, and
c) spray drying said concentrate while maintaining the product temperature at less than 50°C.

The process of the present invention is capable of preserving the ice nucleating activity (INA) after drying of any suspension containing the microorganisms. As noted above, fermentation processes for these microorganisms are well known in the art.

A particularly preferred process is described in European patent application 87113772.5-2105, entitled "Fermentation of Microorganisms Having Ice Nucleating Activity". Further improvements in the fermentation process for this type of microorganism are disclosed in European patent application 87118942.9-2105, entitled "Fermentation of Microorganisms Having Ice Nucleating Activity Using a Defined Medium" and European patent application 88103346.8-2105, entitled "Fermentation of Microorganisms Having Ice Nucleating Activity Using a Temperature Change".

Any microorganism that has ice nucleation activity can be recovered by the present invention. Suitable microorganisms include Pseudomonads such as P. syringae and P. fluorscens, P. coronafaciens and P. pisi. Other microorganisms that are useful in the present invention include Erwinia herbicola. The presently preferred microorganism is P. syringae ATCC No. 53,543, deposited on Sept. 23, 1986 in accordance with the Budapest Treaty with the American Type Culture Collection in Rockville Maryland, USA.

The present method for the recovery of the microorganism results in an acceptable loss of INA on drying while providing a process that is inexpensive and easy to run. In the process of the present invention, it is important to keep the temperature of the microorganisms as low as is practical during the process. The presently preferred process is to first reduce the temperature of the fermentation medium to a temperature below 15°C. The medium is then concentrated to a dry solid by weight concentration of preferably between 15 and 27% and more preferably 22% while keeping the temperature below 15°C.

Concentration of the medium prior to spray drying can be by any conventional method such as filtration or centrifugation. A solid bowl centrifuge or a disc bowl continuous centrifuge can be used.

In accordance with the present invention, the concentrated medium is spray dried so that the product temperature does not exceed 50°C. If the product temperature is maintained at higher temperatures, most of the fermentor INA is lost as is shown in comparative Example 4 below. As the temperature at which the product is maintained is lowered, the product dries more slowly and therefore the through-put for a given spray drying apparatus is lowered. Thus, the preferred temperature range is between 30°C and 50°C.

It was surprising that this temperature sensitive product could be spray dried successfully. In the freeze drying experiments with the previous process, it was noted that the product temperature had to be maintained below about 25°C and still more preferably below 15°C. If the product temperature is maintained this low in a spray drying apparatus, the productivity of the drying is too low to be of significant interest. It was particularly surprising that this product would maintain INA if subjected to drying temperatures in the 30°C to 50°C range.

Any conventional spray drying apparatus can be used to carry out the invention. In a typical spray drying apparatus, the product to be dried is sprayed in the form of small droplets in a chamber containing a large volume of gas. The gas inlet temperature and the flow rate of the product to be dried can be controlled to provide the desired product temperature using well established methods.

The concentrated medium can be atomized in the spray dryer using conventional methods. Pressure nozzles, two fluid nozzles and centrifugal disks, for example, can be used. The gas in the chamber can conveniently be air or it can be some other gas such as nitrogen. Product can be collected using known methods such as cyclone collectors. For a further discussion of the details of spray drying, reference is made to Perry and Chilton eds, Chemical Engineers' Handbook, 5th edition, page 20-58 et seq, McGraw Hill Book Company, 1973.

In the examples presented below, the INA is calculated using conventional techniques. The INA is determined by placing a plurality of microorganism containing water droplets (10 µl) on paraffin coated aluminum foil. The foil is maintained at -5°C by placing it on a constant temperature bath.
Details regarding this procedure are found in the literature, for example, Vali, Quantitative Evaluation of Experimental Results on the Heterogenous Freezing of Sypercooled Liquids, J. Atoms Sci., 28, 402-409 (1971). The INA reported in the examples is the number of ice nucleating sites per dry gram of microorganism. For the present purposes, the INA which is measured using a sample directly from the fermentor without drying will be referred to as "Fermentor INA" and the INA of the recovered dried product will be referred to as the "Recovered INA".

The following examples are submitted for a further understanding of the invention.

### Example 1:

Pseudomonas syringae ATCC 53,543 was streaked on an agar plate containing a nutrient medium containing mannitol, yeast extract and magnesium sulfate. After 48 hours at 26°C, five plates were used to inoculate a 10 liter fermentor also containing a similar medium.

After 12 hours at 26°C this liquid seed was used to inoculate 100 liters of a Fermentation medium, as described in Table I.

**TABLE I**

| Component | Initial Concentration |
|---|---|
| mannitol | 80 g/l |
| yeast extract | 20 g/l |
| magnesium sulfate | 1 g/l |

The fermentation temperature was controlled at 21°C. During the fermentation, the pH was controlled with 4N sulfuric acid and 2N sodium hydroxide. The acid was added when the pH approached 6.6 and the base was added when the pH approached 5.6. The dissolved oxygen was maintained at greater than 30% saturation. The antifoaming agent was added as needed to control foaming.

After 24 hours, the cell mass reached 18 g dry cells/liter. The fermentor INA was 1.51 × 10¹¹.

The fermentation broth was cooled to a temperature of 5°C and centrifuged while the temperature was maintained at 5°C. The solids were collected and slurried to a solids content of 20% in phosphate buffer pH7.0.

The slurry was pumped into a laboratory scale spray dryer made by Niro, Columbia, Maryland, using nitrogen at 118°C. The pump rate was 30 mL/min and the outlet temperature and therefore product temperature was 50°C. The recovered INA was 0.57 × 10¹¹.

### Example 2:

Example 1 was repeated except that the maximum product temperature during spray drying was about 41°C. This was achieved using a gas temperature of 80°C and a pump rate of 20 mL/min. The Recovered INA was 1.29 × 10¹¹.

### Example 3:

Example 1 was repeated except that the maximum product temperature during spray drying was 36°C. This was achieved with a gas temperature of 80°C and a pump rate of 25 mL/min. The Recovered INA was 1.51 × 10¹¹, the same as the Fermentor INA.

### Example 4:

This is a comparative example.

Example 1 was repeated except that the maximum product temperature during spray drying was 68°C. This was achieved with a gas temperature of 118°C and a pump rate of 7 mL/min. The Recovered INA was 0.13 × 10¹¹. This represents less than 10% of the Fermentor INA.

## Claims

1. A method for the recovery of a microorganism which has ice nucleating activity from a fermentation medium said method comprising the steps of:
a) bringing the temperature of said medium to a temperature of 15°C or less,
b) forming a concentrate of said microorganism while maintaining the temperature of 15°C or less, and
c) spray drying said concentrate while maintaining the product temperature at less than 50°C.

2. The method according to claim 1 wherein said microorganism is a Pseudomonad.

3. The method according to claim 2 wherein said microorganism is P. syringae.

4. A method according to claim 1 wherein the product temperature during step c) is maintained between 30°C and 50°C.

## Patentansprüche

1. Verfahren zur Wiedergewinnung eines Mikroorganismus mit Eiskristallbildungsaktivität aus einem Fermentationsmedium mit den Stufen:
a) Einstellen der Temperatur des Mediums auf 15°C oder weniger,
b) Erzeugung eines Konzentrates des Mikroorganismus unter Aufrechterhaltung der Temperatur von 15°C oder weniger, und
c) Sprühtrocknung des Konzentrates unter Aufrechterhaltung der Produkttemperatur bei weniger als 50°C.

2. Verfahren nach Anspruch 1, bei dem der Mikroorganismus ein Pseudomonad ist.

3. Verfahren nach Anspruch 2, bei dem der Mikroorganismus P. syringae ist.

4. Verfahren nach Anspruch 1 , bei dem die Produkttemperatur während der Stufe c) zwischen 30°C und 50°C gehalten wird.

## Revendications

1. Procédé de récupération d'un micro-organisme possédant une activité de nucléation de glace dans un milieu de fermentation, ledit procédé comprenant les étapes consistant à :
a) amener la température dudit milieu à un chiffre inférieur ou égal à 15°C,
b) former un concentré dudit micro-organisme tout en maintenant la température à un chiffre inférieur ou égal à 15°C et
c) sécher par pulvérisation ledit concentré, tout en maintenant la température du produit à un chiffre inférieur à 50°C.

2. Procédé selon la revendication 1, dans lequel ledit micro-organisme est un Pseudomonade.

3. Procédé selon la revendication 2, dans lequel ledit micro-organisme est P. syringae.

4. Procédé selon la revendication 1, dans lequel la température du produit durant l'étape c) est maintenue entre 30°C et 50°C.
